# EUROPÄISCHE PATENTSCHRIFT

(11) **EP 2 656 867 B1**
(45) Veröffentlichungstag und Bekanntmachung des Hinweises auf die Patenterteilung: **15.05.2019**
(21) Anmeldenummer: 13176790.7
(22) Anmeldetag: 04.04.2007
(51) Int. Cl.: A61M 15/00

(54) **Mundstück für einen Inhalator**
Mouthpiece for an inhalator
Embouchure pour un inhalateur

(30) Priorität: 11.04.2006 DE 102006016901
(43) Veröffentlichungstag der Anmeldung: 30.10.2013
(62) Teilanmeldung aus: 07727793.7
(73) Patentinhaber: Boehringer Ingelheim Pharma GmbH & Co. KG, 55216 Ingelheim am Rhein (DE)
(72) Erfinder: Wachtel, Herbert, 55216 Ingelheim am Rhein (DE)
(74) Vertreter: Simon, Elke Anna Maria

(56) Entgegenhaltungen:
- WO-A-94/06498
- WO-A1-98/41259
- WO-A1-2005/102429
- CA-A1- 2 207 219

## Beschreibung

Die Erfindung bezieht sich auf ein Mundstück für einen Inhalator zur Verabreichung von einem Arzneimittel in Form inhalationsfähiger Substanzen, Substanzformulierungen oder -mischungen, das einen Inhalationskanal zur Kopplung mit einer Kammer zur Aufnahme des Arzneimittel aufweist, und einen Inhalator dazu.

Die EP 0 911 047 A1 offenbart einen Inhalator für die Inhalation pulverförmiger Arzneimittel aus Kapseln, der ein Unterteil mit zwei Fenstern und eine Platte, in der sich Kapselhalterungen sowie Lufteinlassöffnungen befinden, umfasst. Im Weiteren ist eine Inhalationskammer mit der Platte verbunden, an der ein mit zwei geschliffenen Nadeln versehener, gegen eine Feder beweglicher Kopf vorgesehen ist. Ein Mundrohr ist mit einem Oberteil des Inhalators und ein Deckel klappbar mit dem Unterteil, der Platte sowie dem Oberteil verbunden.

Des Weiteren beschreibt die DE 39 27 170 A1 einen Inhalator für die Inhalation pulverförmiger, insbesondere mikronisierter Arzneimittel aus Kapseln, in dessen Gehäuse für die Aufnahme der Kapseln eine rohrförmige Kammer mit einem bodenseitigen Lufteinlass und einem in ein Mundstück übergehenden Luftauslass an dem gegenüberliegenden Kammerende und eine Schneideinrichtung mit zwei in dem Kammerinnenraum bewegbaren Schneiden zum Öffnen der Kapseln in der Nähe von deren oberen und unteren Ende angeordnet sind. Im oberen Bereich der Kammer, in der sie in den Inhalationskanal übergeht, ist eine Siebplatte angeordnet, die Teil eines trichterförmigen Verbindungsstückes ist, das auf den Anfang des Inhalationskanals derart aufsteckbar ist, dass der Trichterrand mit der Siebplatte in eine Einsatzplatte eingreift, die den Boden des Mundstückes bildet. Alternativ ist die Siebplatte im Klemmsitz zwischen dem Trichterrand des Verbindungsstückes und einem Anschlag der Einsatzplatte austauschbar befestigt. Dieser Inhalator ist insofern nachteilig, als das Sieb separat zu montieren ist und eine unkontrollierte Verwirbelung des zu inhalierenden Aerosols im Inhalationskanal mit einer damit einhergehenden Deposition des Arzneimittels nicht auszuschließen ist.

Es ist Aufgabe der Erfindung, ein Mundstück und einen Inhalator der eingangs genannten Art zu schaffen, mit denen jeweils eine verbesserte Ausbringung des Arzneimittels gewährleistet ist.

Erfindungsgemäß wird die Aufgabe dadurch gelöst, dass in dem Inhalationskanal mindestens ein Bauteil mit einem aerodynamischen Querschnitt vorgesehen ist.

Aufgrund dieser Maßnahme ist die unkontrollierte Verwirbelung des zu inhalierenden Aerosols im Inhalationskanal verringert und unhygienisch erscheinende Rückstände des Arzneimittels im Bereich des Mundstückes werden verringert. Dem Fachmann sind aus der Strömungstechnik hinreichend Profile mit einem aerodynamischen Querschnitt bekannt, mit denen sich eine verhältnismäßig geringe Verwirbelung eines Aerosols beim Inhalieren realisieren lässt.

Inhalatoren sind unter den Markennamen HandiHaler®, Spinhaler®, Rotahaler®, Aerolizer®, Flowcaps®, Turbospin®, AIR DPI®, Orbital®, Directhaler® bekannt und/oder in DE 33 45 722, EP 0 591 136, DE 43 18 455, WO 91/02558, FR-A-2 146 202, US-A-4 069 819, EP 666085, EP 869079, US 3,991,761, WO 99/45987, WO 200051672, Bell, J. Pharm. Sci. 60, 1559 (1971); Cox, Brit. Med. J. 2, 634 (1969), beschrieben. Als Pulverinhalatoren sind Einfach- oder Mehrfachdosis-Pulverinhalatoren, insbesondere der Spinhaler®, Rotahaler®, Aerolizer®, Inhalator®, HandiHaler®, Diskhaler®, Diskus®, Accuhaler®, Aerohaler®, Eclipse®, Turbohaler®, Turbuhaler®, Easyhaler®, Novolizer®, Clickhaler®, Pulvinal®, Novolizer®, SkyeHaler®, Xcelovair®, Pulvina®, Taifun®, MAGhaler®, Twisthaler® und der Jethaler® bekannt.

Die unten genannten Verbindungen können allein oder in Kombination zur Anwendung in der erfindungsgemäßen Vorrichtung gelangen. In den unten genannten Verbindungen ist W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmern oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-(1-hydroxy-2-[2-(4-methoxy-phenyl)-1,1-dimethyl-ethylamino]-ethyl)-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1 ,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-(2-(4-hydroxy-phenyl)-1,1-dimethyl-ethylamino]-ethyl}-4H-benzo[1,4]oxazin-3-on
- 6-Hydroxy-8-{ 1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-[2-[4-(6-methoxy-biphenyl-3-ylamino)-phenyl]-ethylamino }-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinotin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 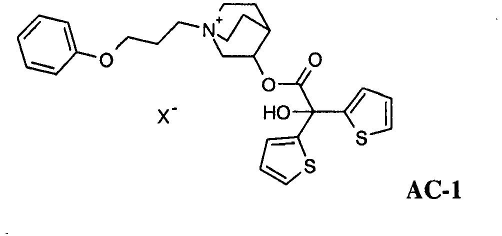 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 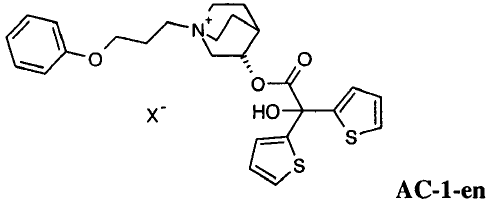 enthalten, worin X⁻ die vorstehend genannten Bedeutungen aufweisen kann. Weiterhin bevorzugte Anticholinergika sind ausgewählt aus den Salzen der Formel **AC-2** 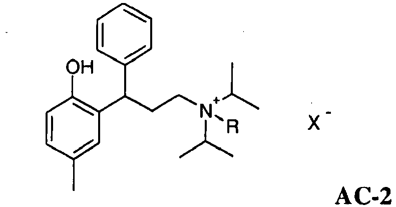 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC-2-base** vorliegen.

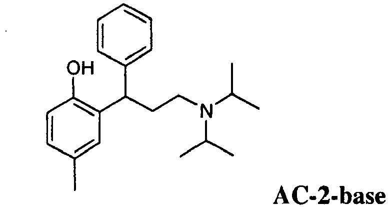

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3 '-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxyl-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure(S)-(2-oxo-tetrahydro-furan-3S-yl)ester,
- 6α,9α-difluoro-11β-hydroxy-16α-methyl-3-oxo-17α-(2,2,3,3-tertamethylcyclo-propylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, A-riflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{ [4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino} -7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino }-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten- 1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-aminol-1-oxo-2-buten-l-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)aminol-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[l-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{ 1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Für die Inhalation kommen Arzneimittel mit den o.g. Wirkstoffen in Betracht, sowie deren Salze, Ester sowie die Kombination dieser Wirkstoffe, Salze und Ester.

Bevorzugt ist das Bauteil einstückig mit dem Inhalationskanal verbunden und erstreckt sich radial in den Inhalationskanal. Damit lassen sich Kosten bei der Montage des Mundstückes bzw. Inhalators einsparen.

Nach einer Weiterbildung ist das Bauteil als Stromlinienkörper, insbesondere NACA-Profil, ausgebildet. Bei einem NACA-Profil handelt es sich um ein symmetrisches Profil einer Versuchsreihe, die das National Advisory Commitee for Aerodynamic (NACA) durchgeführt hat. NACA-Profile zeichnen sich durch eine abgerundete Nase und ein spitz auslaufendes hinteres Teil aus. Unterschiedliche NACA-Profile sind bespielsweise in Dubs, Fritz, Aerodynamik der reinen Unterschallströmung, 4. neubearb. Auflage, Birkhäuser Verlag Basel, ISBN 3-7643-1073-1, auf den Seiten 210 bis 213, 218, 219, 222 und 223 beschrieben.

Vorzugsweise wird ein NACA-Profil 0030 oder ein ähnliches Profil verwendet, wobei sich zeigte, dass die abgegebene Dosis des Arzneimittels durch die Verwendung eines solchen Profils gegenüber einem konventionellen Sieb vergrößert ist. Zweckmäßigerweise weist die abgerundete Nase des Stromlinienkörpers in Richtung des Mundstücks und das spitze Ende zur Kammer.

Um zu verhindern, dass relativ große Partikel duch den Inhalationskanal gelangen und darüber hinaus einen Anschlag für eine das Arzneimittel aufnahmende Kapsel bereitzustellen, sind mehrere Stromlinienkörper sternförmig zueinander ausgerichtet. Durch diese Anordnung ist auch eine verhältnismäßig hohe Stabilität gewährleistet, die zur Entleerung der bei der Inhalation vibrierenden Kapsel beiträgt. Im Weiteren lässt sich mit der sternförmigen Ausrichtung der NACA-Profile ein zyklonartiger Wirbel beim Inhalieren erzeugen, der zur Modifikation der Teilchengrößenverteilunng des Arzneimittels eingesetzt werden kann, da ein starker Wirbel zu einer bevorzugten Deposition schwerer und großer Partikel im Inhalationskanal führt, während leichte Wirkstoffteilchen zum Patienten bzw. Benutzer des Inhalators gelangen. Bevorzugt ist hierbei der Inhalationskanal im Querschnitt rund ausgebildet. Zweckmäßigerweise sind die Stromlinienkörper an dem der Mundseite gegenüberliegenden Seite des Inhalationskanals angeordnet.

In Ausgestaltung besteht das Mundstück mitsamt dem Inhalationskanal und den NACA-Profilen aus einem Kunststoff. Vorzugsweise handelt es sich bei den Kunststoffen um Polymerisate, thermoplastische Polykondensate, Polyaddukte, abgewandelte Naturstoffe oder Kautschuke bzw. um Gemische dieser Kunststoffe. Besonders bevorzugt sind hier Polyolefine, Vinylchlorid-Polymerisate, Styrol-Polymerisate, Polyacetale, Polyamide, thermoplastische Polyester und Polyarylether bzw. Gemische dieser Kunststoffe. Beispiele für diese Kunststoffe sind Polyethylen, Polyvinylchlorid, Polyoxymethylen, Polyacetal, Acrylnitril/Butadien/Styrol(ABS), Acrylnitril/Styrol/Acryl-ester(ASA), Polyamide, Polycarbonat, Poly (ethylenterephthalat), Poly(butylenterephthalat) oder Poly(phenylenether). Derartige Kunststoffe können beispielsweise von der Firma Ensinger in Deutschland, Nufringen, bezogen werden.

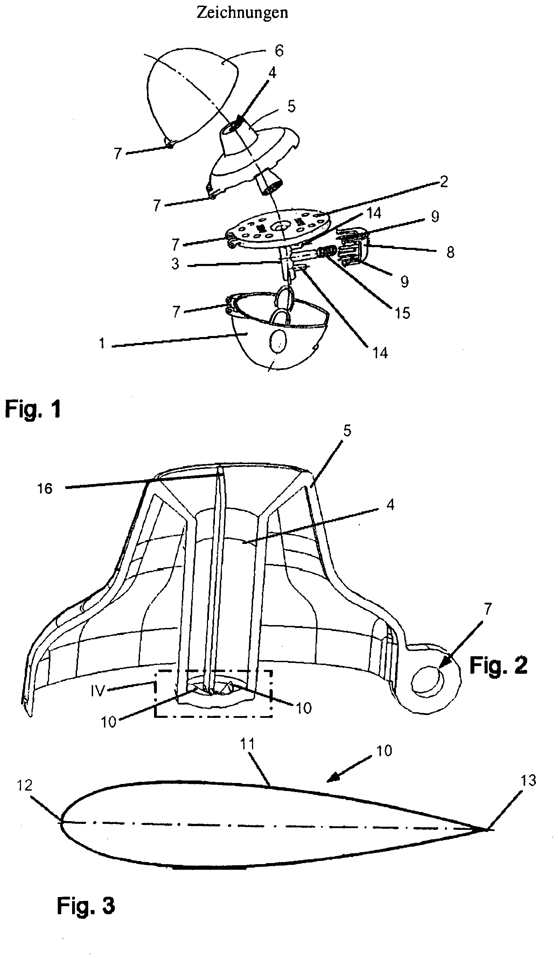

Vorzugsweise ist das Mundstück mitsamt dem Inhalationskanal und den darin angeordneten NACA-Profilen im Spritzgussverfahren herstellbar. Die NACA-Profile dienen hierbei als Verteiler für die Kunststoffschmelze, wobei ein Anspritzpunkt beispielsweise im Zentrum der sternförmig zueinander angeordneten Profile ausgebildet sein kann. Außer einer sternförmigen Anordnung ist in einer weiteren Ausführungsform auch eine bügelartige Verbindung der Stege im Mundstückinnenkanal möglich (Fig. 2).

Das zuvor beschriebene Mundstück findet bevorzugt in einem Inhalator für die Inhalation eines pulverförmigen Arzneimittels aus einer Kapsel Verwendung, wobei der Inhalator ein Unterteil umfasst, an dem eine mit dem Unterteil verrastbare Platte zum Verschließen des Unterteils angelenkt ist. Die Platte weist eine in dem Unterteil versenkbare Kapselhalterung zur Aufnahme der Kapsel auf und ist mit dem Mundstück verrastbar. Ein Deckel, der das Mundstück in einer Verschlussposition abdeckt, ist mittels eines Verschlusselementes verrastet. Das Unterteil, die Platte, das Mundstück und der Deckel sind durch ein einziges Gelenk verschwenkbar miteinander verbunden. Darüber hinaus ist ein Betätigungsorgan vorgesehen, das aus einer Ruhestellung in eine Bewegung versetzbar ist und dabei mit mindestens einer in die Kapselhalterung einstoßbaren Nadel zusammenwirkt. Bevorzugt sind zwei zueinander beabstandete Nadeln vorgesehen.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispieles unter Bezugnahme auf die zugehörigen Zeichnungen näher erläutert. Es zeigt:
- Fig.1: eine perspektivische Teil-Schnittdarstellung eines Inhalators mit einem erfindungsgemäßen Mundstück,
- Fig.2: eine perspektivische Darstellung eines Schnittes durch das Mundstück nach Fig. 1,
- Fig.3: einen Querschnitt durch ein aerodynamisches Bauteil des Mundstücks gemäß der Darstellung nach Fig. 2 und

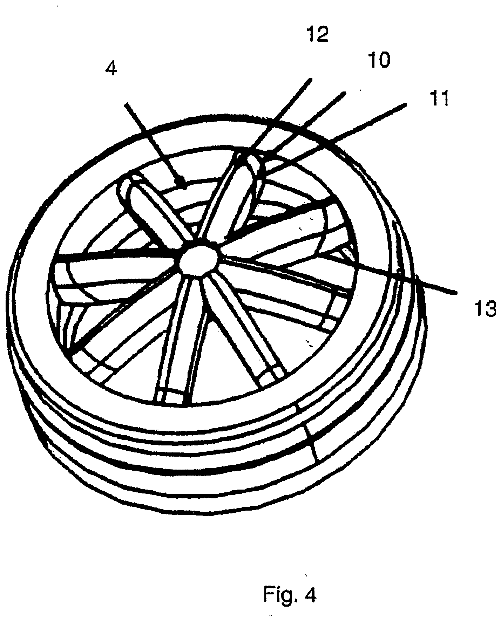

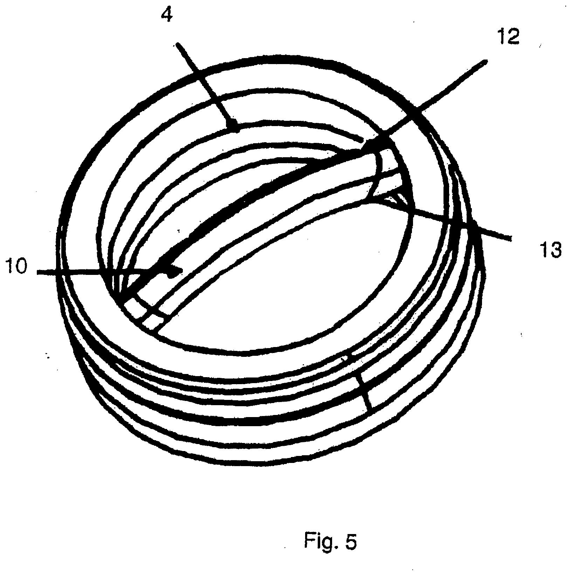

- Fig.4: eine vergrößerte perspektivische Darstellung der Einzelheit IV nach Fig. 2.

Der Inhalator weist ein Unterteil 1 auf, an dem eine mit dem Unterteil 1 verrastbare Platte 2 zum Verschließen des Unterteils 1 angelenkt ist. Die Platte 2 ist mit einer in dem Unterteil 1 versenkbaren Kapselhalterung 3 zur Aufnahme einer mit einem pulverförmigen Arzneimittel gefüllten Kapsel in einer Kammer versehen und mit einem einen Inhalationskanal 4 aufweisenden Mundstück 5 verrastbar. Ein Deckel 6, der das Mundstück 5 in einer Verschlussposition abdeckt, ist mittels eines Verschlusselementes verrastet. Das Unterteil 1, die Platte 2, das Mundstück 5 und der Deckel 6 sind durch ein gemeinsames Gelenk 7 verschwenkbar miteinander verbunden. Darüber hinaus ist ein Betätigungsorgan 8 vorgesehen, das mit zwei in die Kapselhalterung 3 einstoßbaren Nadeln 9 zum Einstechen der Kapsel zusammenwirkt. Die zueinander beabstandeten Nadeln 9 sind in Führungsstutzen 14 der Kapselhalterung 3 verschiebbar gelagert. Um eine Rückstellbewegung der Nadeln 9 zur Freigabe der Löcher in der Kapsel nach dem Loslassen des Betätigungsorgans 8 zu bewirken, ist zwischen der Kapselhalterung 3 und dem Betätigungsorgan 8 eine Druckfeder 15 angeordnet.

Das Mundstück 5 besteht mit dem darin ausgebildeten im Querschnitt runden Inhalationkanal 4 aus einem Kunststoff und ist im Spritzgussverfahren gefertigt. An dem der Mundseite gegenüberliegenden Seite des Inhalationskanals 4 sind mehrere sternförmig zueinander ausgerichtete Bauteile 10 mit einem aerodynamischen Querschnitt angeordnet. Die Bauteile 10 sind als NACA-Profile 11 ausgeführt und weisen jeweils eine abgerundete Nase 12 sowie ein spitzes Ende 13 auf, wobei das Ende auch abgerundet sein kann, um die Herstellung zu vereinfachen. Das Mundstück besitzt außerdem einen oder mehrere Stege 16, die das missbräuchliche Einführen von nicht geeigneten Kapseln verhindern, weiterhin das Strömungsverhalten des Inhalators und die Austrittsgeschwindigkeit des Arzneimittels günstig beeinflussen.

## Patentansprüche

1. Inhalator zur Verabreichung eines pulverförmigen Arzneimittels in Form inhalationsfähiger Substanzen, Substanzformulierungen oder -mischungen aus einer Kapsel, wobei der Inhalator eine Kapselhalterung (3) zur Aufnahme der mit dem Arzneimittel gefüllten Kapsel in einer rohrförmigen Kammer und ein Mundstück (5) aufweist, wobei die Kapsel zur Entleerung bei der Inhalation vibriert, und wobei das Mundstück (5) einen Inhalationskanal (4) zur Kopplung mit der Kammer aufweist und die Kammer im oberen Bereich in den Inhalationskanal übergeht, und wobei der Inhalator ein Betätigungsorgan (8) aufweist, das aus einer Ruhestellung in eine Bewegung versetzbar ist und dabei mit mindestens einer in die Kapselhalterung (3) einstoßbaren Nadel (9) zum Einstechen der Kapsel zusammenwirkt,
**dadurch gekennzeichnet, dass**
das Mundstück mindestens einen Steg (16) aufweist, der das missbräuchliche Einführen von Kapseln verhindert.

2. Inhalator nach Anspruch 1, **dadurch gekennzeichnet, dass** der Inhalationskanal (4) im Querschnitt rund ausgebildet ist und sich der Steg (16) an der Innenwand des Inhaltionskanals (4) befindet.

3. Inhalator nach Anspruch 1 oder 2 **dadurch gekennzeichnet, dass** in dem Inhalationskanal (4) mindestens ein Bauteil (10) mit einem aerodynamischen Querschnitt vorgesehen ist.

4. Inhalator nach Anspruch 3, **dadurch gekennzeichnet, dass** das Bauteil (10) einstückig mit dem Inhalationskanal (4) verbunden ist und sich radial in den Inhalationskanal (4) erstreckt.

5. Inhalator nach Anspruch 3 oder 4, **dadurch gekennzeichnet, dass** das Bauteil (10) als Stromlinienkörper ausgebildet ist.

6. Inhalator nach Anspruch 5, **dadurch gekennzeichnet, dass** der Stromlinienkörper ein NACA-Profil (11) mit einer abgerundeten Nase (12) und einem spitzen Ende (13) aufweist und die abgerundete Nase (12) des Stromlinienkörpers in Richtung des Mundstücks (5) und das spitze Ende (13) zur Kammer weist.

7. Inhalator nach Anspruch 5 oder 6, **dadurch gekennzeichnet, dass** mehrere Stromlinienkörper sternförmig zueinander ausgerichtet sind.

8. Inhalator nach einem der Ansprüche 5 bis 7, **dadurch gekennzeichnet, dass** die Stromlinienkörper an dem der Mundseite gegenüberliegenden Seite des Inhalationskanals (4) angeordnet sind.

9. Inhalator nach einem der Ansprüche 1 bis 8, **dadurch gekennzeichnet, dass** das Mundstück (5) aus einem Kunststoff besteht.

10. Inhalator nach einem der Ansprüche 1 bis 9, **dadurch gekennzeichnet, dass** das Mundstück (5) im Spritzgussverfahren herstellbar ist.

11. Inhalator nach einem der Ansprüche 1 bis 10 zur Applikation einer pulverförmigen Arzneimittels mit einem Wirkstoff aus der Gruppe der Anticholinergika, Betamimetika, Steroide, Phosphodiesterase IV-inhibitoren, LTD4-Antagonisten, EGFR-Kinase-Hemmer, Antiallergika, Derivate von Mutterkornalkaloiden, Triptane, CGRP-Antagonisten, Phosphodiesterase-V-Inhibitoren, sowie Kombinationen aus solchen Wirkstoffen.

## Claims

1. Inhaler for administering a powdered medicament in the form of inhalable substances, substance formulations or substance mixtures from a capsule, the inhaler comprising a capsule holder (3) for receiving the capsule filled with the medicament in a tubular chamber and a mouthpiece (5),
wherein for emptying, the capsule vibrates during inhalation, and
wherein the mouthpiece comprises an inhalation channel (4) for coupling to the chamber and the chamber merges into the inhalation channel in the upper part of the chamber,and
wherein the inhaler comprises an actuator (8) that can be shifted into movement from an inactive position and in the process interacts with at least one needle (9) that can be pushed into the capsule holder (3) for piercing the capsule,
**characterised in that**
the mouthpiece comprises at least one bar (16) which prevents the improper insertion of capsules.

2. Inhaler according to claim 1, **characterised in that** the inhalation channel (4) is round in cross section and said bar (16) is located on the inner wall of the inhalation channel (4).

3. Inhaler according to claim 1 or 2, **characterised in that** at least one component (10) having an aerodynamic cross section is provided in the inhalation channel (4).

4. Inhaler according to claim 3, **characterized in that** said component (10) is integrally connected to the inhalation channel (4) and extends radially into the inhalation channel (4).

5. Inhaler according to claim 3 or 4, **characterized in that** said component (10) is designed as a streamlined body.

6. Inhaler according to claim 5, **characterised in that** the streamlined body comprises a NACA-profile (11) with a rounded nose (12) and a pointed end (13) and the rounded nose (12) of the streamlined body points towards the mouthpiece (5) and the pointed end (13) points towards the chamber.

7. Inhaler according to claim 5 or 6, **characterised in that** a plurality of streamlined bodies are orientated with respect to one another so as to form a star.

8. Inhaler according to any one of claims 5 to 7, **characterised in that** the streamlined bodies are arranged at the end of the inhalation channel (4) that is opposite the mouth end.

9. Inhaler according to any one of claims 1 to 8, **characterised in that** the mouthpiece (5) consists of a plastics material.

10. Inhaler according to any one of claims 1 to 9, **characterised in that** the mouthpiece (5) can be produced by an injection moulding process.

11. Inhaler according to any one of claims 1 to 10 for applying a powdered medicament having an active ingredient from the group consisting of anticholinergics, betamimetics, steroids, phosphodiesterase-4 inhibitors, LTD4 antagonists, EGFR kinase inhibitors, anti-allergic agents, ergot alkaloid derivatives, triptans, CGRP antagonists, phosphodiesterase-5 inhibitors, and combinations of active ingredients of this kind.

## Revendications

1. Inhalateur pour l'administration d'un médicament sous forme pulvérulente sous la forme de substances pouvant être inhalées, de formulations ou de mélanges de substances d'une capsule, dans lequel l'inhalateur présente un support de capsule (3) servant à recevoir la capsule remplie du médicament dans une chambre tubulaire et un embout buccal (5), dans lequel la capsule vibre lors de l'inhalation aux fins du déchargement, et dans lequel l'embout buccal (5) présente un canal d'inhalation (4) pour le couplage avec la chambre et la chambre s'imbrique dans la zone supérieure dans le canal d'inhalation, et dans lequel l'inhalateur présente un organe d'actionnement (8), qui peut être déplacé d'une position de repos en un mouvement et coopère ainsi avec au moins une aiguille (9) pouvant être introduite dans le support de capsule (3), servant à transpercer la capsule,
**caractérisé en ce que**
l'embout buccal présente au moins une traverse (16), qui empêche l'introduction abusive de capsules.

2. Inhalateur selon la revendication 1, **caractérisé en ce que** le canal d'inhalation (4) est réalisé de manière arrondie dans la section transversale et la traverse (16) se trouve au niveau de la paroi intérieure du canal d'inhalation (4).

3. Inhalateur selon la revendication 1 ou 2, **caractérisé en ce qu'**au moins un composant (10) avec une section transversale aérodynamique est prévu dans le canal d'inhalation (4).

4. Inhalateur selon la revendication 3, **caractérisé en ce que** le composant (10) est relié d'un seul tenant au canal d'inhalation (4) et s'étend de manière radiale dans le canal d'inhalation (4).

5. Inhalateur selon la revendication 3 ou 4, **caractérisé en ce que** le composant (10) est réalisé sous la forme d'un profil aérodynamique.

6. Inhalateur selon la revendication 5, **caractérisé en ce que** le profil aérodynamique présente un profil NACA (11) avec un nez (12) arrondi et une extrémité (13) pointue, et le nez (12) arrondi du profil aérodynamique pointe en direction de l'embout buccal (5) et l'extrémité (13) pointue pointe en direction de la chambre.

7. Inhalateur selon la revendication 5 ou 6, **caractérisé en ce que** plusieurs profils aérodynamiques sont orientés les uns par rapport aux autres de manière à présenter une forme d'étoile.

8. Inhalateur selon l'une quelconque des revendications 5 à 7, **caractérisé en ce que** les profils aérodynamiques sont disposés sur le côté, opposé au côté buccal, du canal d'inhalation (4).

9. Inhalateur selon l'une des revendications 1 à 8, **caractérisé en ce que** l'embout buccal (5) est constitué d'une matière plastique.

10. Inhalateur selon l'une des revendications 1 à 9, **caractérisé en ce que** l'embout buccal (5) peut être fabriqué dans un procédé de coulage par injection.

11. Inhalateur selon l'une des revendications 1 à 10, pour l'application d'un médicament sous forme pulvérulente ayant un principe actif du groupe des anticholinergiques, des bêta-mimétiques, des stéroïdes, des inhibiteurs de la phosphodiestérase IV, des antagonistes de LTD4, des inhibiteurs de l'EGFR-kinase, des antiallergiques, des dérivés d'alcaloïdes de l'ergot, de triptans, d'antagonistes de CGRP, d'inhibiteurs de la phosphodiestérase V ainsi que des combinaisons de ces principes actifs.
